(19) Europäisches Patentamt / European Patent Office / Office européen des brevets

(11) **EP 3 098 508 B1**

(12) **EUROPEAN PATENT SPECIFICATION**

(45) Date of publication and mention
of the grant of the patent:
**30.03.2022 Bulletin 2022/13**

(21) Application number: **14875774.3**

(22) Date of filing: **20.08.2014**

(51) International Patent Classification (IPC):
***F22B 37/38*** *(2006.01)*

(52) Cooperative Patent Classification (CPC):
**F22B 37/38**

(86) International application number:
**PCT/JP2014/071811**

(87) International publication number:
**WO 2015/098179 (02.07.2015 Gazette 2015/26)**

(54) **HEAT TRANSFER TUBE LIFE ESTIMATING SYSTEM**

SYSTEM ZUR LEBENSDAUERSCHÄTZUNG EINES WÄRMETRANSFERROHRS

SYSTÈME D'ESTIMATION DE LA DURÉE DE VIE D'UN TUBE DE TRANSFERT DE CHALEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.12.2013 JP 2013270942**

(43) Date of publication of application:
**30.11.2016 Bulletin 2016/48**

(73) Proprietor: **Kawasaki Jukogyo Kabushiki Kaisha Kobe-shi, Hyogo 650-8670 (JP)**

(72) Inventors:
• **OZAKI Daisuke**
  **Kobe-shi, Hyogo 650-8670 (JP)**
• **EMA Shigeyuki**
  **Tokyo 105-8315 (JP)**

• **TODA Shinichi**
  **Kobe-shi, Hyogo 650-8670 (JP)**
• **IMAI Tatsuya**
  **Akashi-shi, Hyogo 673-8666 (JP)**
• **SHIRATSUCHI Toru**
  **Akashi-shi, Hyogo 673-8666 (JP)**

(74) Representative: **AWA Sweden AB
Junkersgatan 1
582 35 Linköping (SE)**

(56) References cited:
JP-A- H06 331 622     JP-A- H08 285 211
JP-A- H09 119 603     JP-A- 2000 292 419
JP-A- 2002 022 686    JP-A- 2011 064 381
JP-A- 2013 185 764    KR-A- 20110 015 258

EP 3 098 508 B1

## Description

Technical Field

The present invention relates to a heat transfer tube life estimating system for estimating life of a heat transfer tube such as a furnace wall water tube of a boiler.

Background Art

A heat transfer tube such as a furnace wall water tube of a boiler becomes high in temperature during operation, and therefore it is necessary to monitor a creep damage state by heat of a heat transfer tube material. Therefore, conventionally, part of a heat transfer tube material is cut and observed with a microscope in a periodic inspection so as to inspect the creep damage state during operation stoppage of a boiler.

A membrane panel (boiler water-cooled wall) configuring a boiler furnace wall is heated by burner flame or combustion gas from the inner side of the furnace and exchanges heat with water and/or steam flowing through its cooling tube (water tube). In other words, it is a state that a metal tube heated by flame or combustion gas is cooled by water.

Also, scale is accumulated in a cooling tube of the membrane panel gradually according to the operation state of a boiler (temperature, time, and the like). As heat conductivity of scale is inefficient, when scale is adhered to the inside of the cooling tube, heat transfer efficiency of the cooling tube declines according to its adhesion quantity.

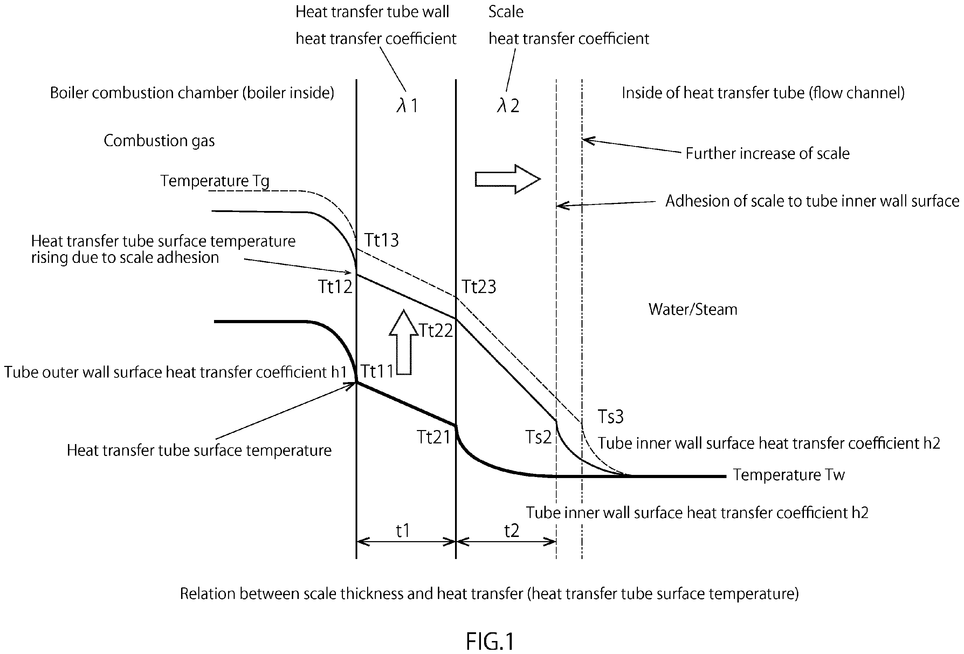

It is illustrated in FIG. 1 giving an evaporator tube as an example. For example, provided that the heat conductivity of scale is constant, a temperature difference inside scale increases and a temperature of a surface of the heat transfer tube rises according to increase of adhesion thickness of scale. A heat transfer tube surface temperature is $Tt_{22}$ [K] , a fluid temperature is Tw [K] , a scale surface heat conductivity is $h_2$ [W/m$^2$ • K] , a thickness and a heat conductively of scale are $t_2$ [m] and $\lambda_2$ [w/m • K] , respectively, and then a heat flux q [W/m$^2$] is represented by the formula below.

$$\mathrm{q} = \boldsymbol{h_e}\triangle\mathrm{T}$$

Here, $\boldsymbol{h_e}$=1/($t_2$/$\lambda_2$+1/$h_2$) : equivalent heat transfer coefficient [W/m$^2$ • K], $\Delta T=Tt_{22}$-Tw[K] is established.

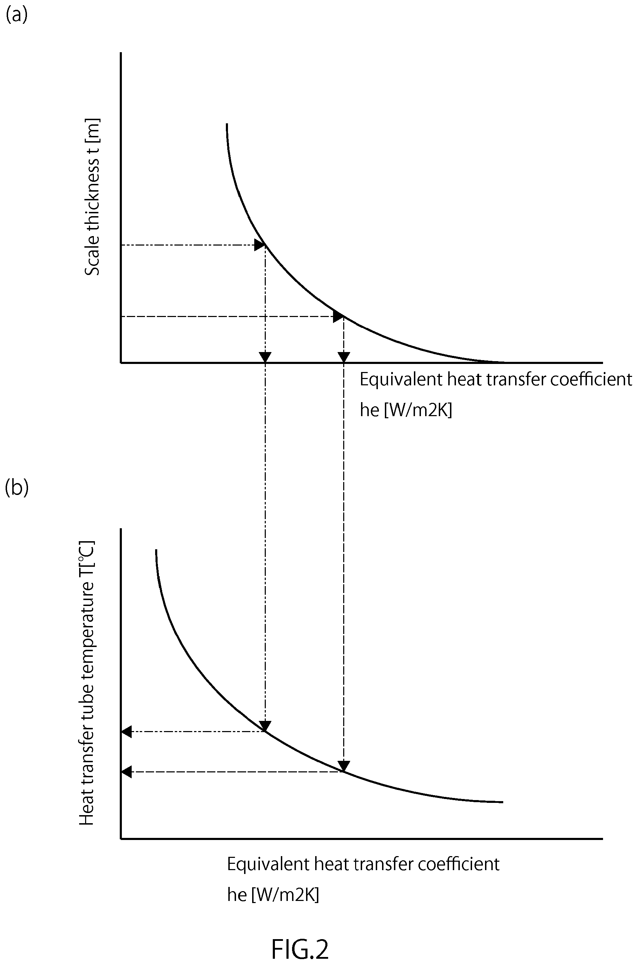

Also, FIG. 2 (a) illustrates a relation between a scale thickness and an equivalent heat transfer coefficient, and FIG. 2(b) illustrates a relation between a heat transfer tube temperature and the equivalent heat transfer coefficient. From the figures, it can be seen that the equivalent heat transfer coefficient increases as the scale thickness increases, and thereby the heat transfer tube temperature increases as a result.

Note that the equivalent heat transfer coefficient is a function of scale thickness and also heat conductivity of scale, and therefore it changes also by material and adhesion state (density, etc.) of scale. Further, as a surface heat conductivity $h_2$ changes by a scale surface state, the equivalent heat transfer coefficient changes by the change as well.

Thereby, for example, when a vicious circle of heating/scale growth by abnormal combustion or the like in a furnace and metal temperature increase by heat transfer deterioration is accelerated, there is a possibility that the metal temperature of the cooling tube increases more quickly than an estimated period and the tube is broken by a high-temperature creep, thereby stopping the boiler. Therefore, it is important to precisely grasp the accumulation of creep damage of the cooling tube of the membrane panel. JP 2002 022686 A discloses a method for evaluating damage and stain of high temperature heat transfer part.

For example, damage rate predicting method of a boiler heat transfer tube material is proposed in PTL 1.

Citation List

Patent Literature

[PTL 1] Japanese Patent Application Laid-Open No. 2005-

Summary of Invention

Technical Problem

However, in a conventional technique, the creep life-time of the heat transfer tube of the boiler cannot be monitored during boiler operation in real time. Therefore, there is a problem that maintenance or repair cannot be performed timely.

The present invention is made considering the above-mentioned problems of the conventional technique and its object is to provide a heat transfer tube life estimating system capable of estimating the life of a heat transfer tube of a boiler while the boiler is in operation in real time.

Solution to Problem

A heat transfer tube life estimating system according to the invention is described in claim 1.

In order to achieve the object above, the present invention comprises, in a heat transfer tube life estimating system for estimating a life of a heat transfer tube of a boiler, a heat transfer tube temperature time change curve calibrating means for calibrating a heat transfer tube temperature time change curve representing a change in the heat transfer tube temperature with time based on the heat transfer tube temperature as a temperature of an outer wall surface of the heat transfer tube, and a heat transfer tube temperature transition predicting means for predicting transition in the heat transfer tube temperature based on the heat transfer tube temperature time change curve which has been calibrated.

Also, the present invention comprises, in a heat transfer tube life estimating system for estimating a life of a heat transfer tube of a boiler, an equivalent heat transfer coefficient calculating means for calculating an equivalent heat transfer coefficient inside of the heat transfer tube based on a heat transfer tube temperature as a temperature of an outer wall surface of the heat transfer tube, an equivalent heat transfer coefficient time change curve calibrating means for calibrating an equivalent heat transfer coefficient time change curve representing a change in the equivalent heat transfer coefficient with time based on the equivalent heat transfer coefficient, and a heat transfer tube temperature transition predicting means for predicting transition in the heat transfer tube temperature based on the equivalent heat transfer coefficient time change curve which has been calibrated.

Further, the present invention comprises, in a heat transfer tube life estimating system for estimating a life of a heat transfer tube of a boiler, an equivalent heat transfer coefficient calculating means for calculating an equivalent heat transfer coefficient inside of the heat transfer tube based on a heat transfer tube temperature as a temperature of an outer wall surface of the heat transfer tube, a scale thickness calculating means for calculating a thickness of a scale adhered to an inner wall surface of the heat transfer tube based on the equivalent heat transfer coefficient, a scale thickness time change curve calibrating means for calibrating a scale thickness time change curve representing a change in the scale thickness with time based on the thickness of a scale, and a heat transfer tube temperature transition predicting means for predicting transition in the heat transfer tube temperature based on the scale thickness time change curve which has been calibrated.

Also, it is preferable that a creep life-time predicting means for predicting a creep life-time of the heat transfer tube based on a transition predicting data of the heat transfer tube temperature obtained by the heat transfer tube temperature transition predicting means is further provided.

Also, it is preferable that the heat transfer tube is a cooling tube of a membrane panel configuring a furnace wall of the boiler, wherein an out-furnace fin temperature measuring means for measuring an out-furnace fin temperature as a temperature of an out-furnace side outer wall surface of a fin of the membrane panel and an in-furnace cooling tube temperature calculating means for calculating an in-furnace cooling tube temperature as a temperature of an in-furnace side outer wall surface of the cooling tube based on the out-furnace fin temperature are further provided, and wherein the in-furnace cooling tube temperature is used as the heat transfer tube temperature.

Also, it is preferable that the heat transfer tube temperature is continuously obtained along an axial direction of the heat transfer tube.

Also, it is preferable that the heat transfer tube temperature is obtained over an entire surface of a furnace wall of the boiler.

Also, it is preferable that the heat transfer tube temperature is obtained using an optical fiber or an infrared camera.

Advantageous Effect of Invention

According to the present invention, the life of a heat transfer tube of a boiler can be estimated while the boiler is in operation in real time.

Brief Description of the Drawings

FIG. 1 illustrates heat transfer of a heat transfer tube of a boiler.
FIG. 2(a) illustrates the relation between a scale thickness of the heat transfer tube of the boiler and an equivalent heat transfer coefficient, and (b) illustrates the relation between a heat transfer tube temperature of the heat transfer tube of the boiler and the equivalent heat transfer coefficient.

FIG. 3 is a block diagram illustrating a schematic configuration of a heat transfer tube life estimating system according to an example not covered by the present invention.

FIG. 4 a schematic view illustrating an example of a heat transfer tube temperature measuring means in the heat transfer tube life estimating system illustrated in FIG. 3.

FIG. 5 is a schematic view illustrating an example of the heat transfer tube temperature measuring means in the heat transfer tube life estimating system illustrated in FIG. 3.

FIG. 6 is a schematic view illustrating an example of the heat transfer tube temperature measuring means in the heat transfer tube life estimating system illustrated in FIG. 3.

FIG. 7 is a schematic view illustrating an example of an out-furnace fin temperature measuring means and an in-furnace cooling tube temperature calculating means in the heat transfer tube life estimating system in FIG. 3.

FIG. 8 is a chart illustrating an example of an in-furnace cooling tube temperature calculating means in the heat transfer tube life estimating system in FIG. 3.

FIG. 9 is a chart illustrating an example of a heat transfer tube temperature time change curve calibrating means in the heat transfer tube life estimating system in FIG. 3.

FIG. 10 is a chart illustrating an example of the heat transfer tube temperature time change curve calibrating means in the heat transfer tube life estimating system in FIG. 3.

FIG. 11 is a chart illustrating an example of a creep life-time predicting means in the heat transfer tube life estimating system in FIG. 3.

FIG. 12 is a schematic view illustrating an example of a method for displaying the prediction result of the creep life-time predicting means in the heat transfer tube life estimating system in FIG. 3.

FIG. 13 is a block diagram illustrating a schematic configuration of the heat transfer tube life estimating system according to an embodiment of the present invention.

FIG. 14 is a chart illustrating an example of an equivalent heat transfer coefficient calculating means in the heat transfer tube life estimating system in FIG. 13.

FIG. 15 is a chart illustrating an example of the equivalent heat transfer coefficient time change curve calibrating means in the heat transfer tube life estimating system in FIG. 13.

FIG. 16 is a chart illustrating an example of the equivalent heat transfer coefficient time change curve calibrating means in the heat transfer tube life estimating system in FIG. 13.

FIG. 17 is a block diagram illustrating a schematic configuration of the heat transfer tube life estimating system according to an example not covered by the present invention.

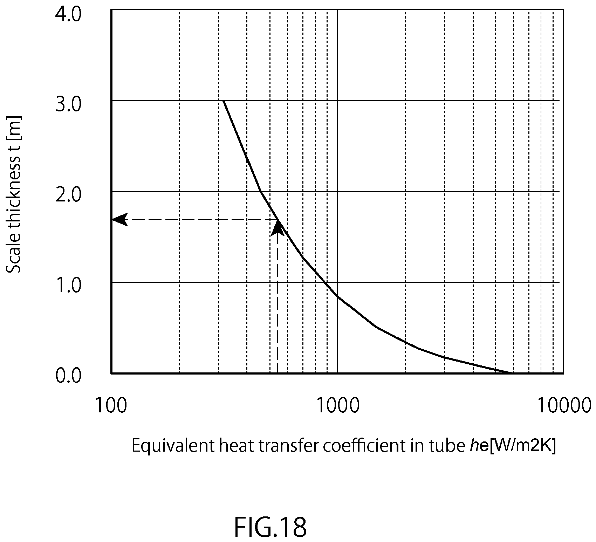

FIG. 18. is a chart illustrating an example of a scale thickness calculating means in the heat transfer tube life estimating system in FIG. 17.

FIG. 19. is a chart illustrating an example of the scale thickness time change curve calibrating means in the heat transfer tube life estimating system in FIG. 17.

FIG. 20. is a chart illustrating an example of the scale thickness calculating means in the heat transfer tube life estimating system in FIG. 17.

Description of Embodiments

A heat transfer tube life estimating system according to a first embodiment of the present invention will be described referring to the figures. The heat transfer tube life estimating system according to the embodiment estimates the life of a heat transfer tube such as a water tube of a boiler's furnace wall (membrane panel), an overheating tube, or the like.

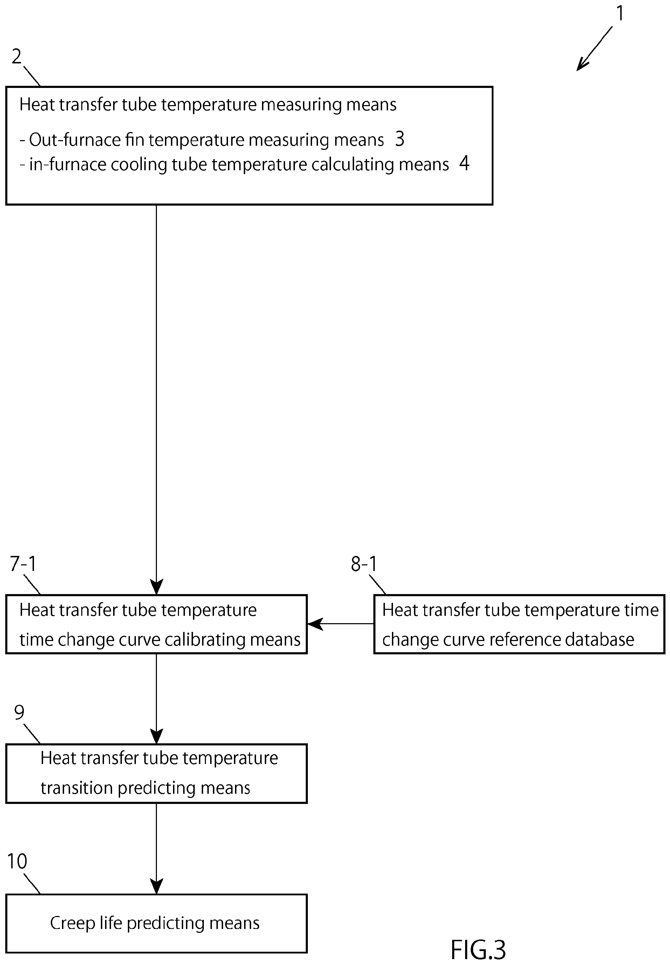

As illustrated in FIG. 3, a heat transfer tube life estimating system 1 according to an example comprises a heat transfer tube temperature measuring means 2 for measuring a heat transfer tube temperature as a temperature of an outer wall surface of a heat transfer tube.

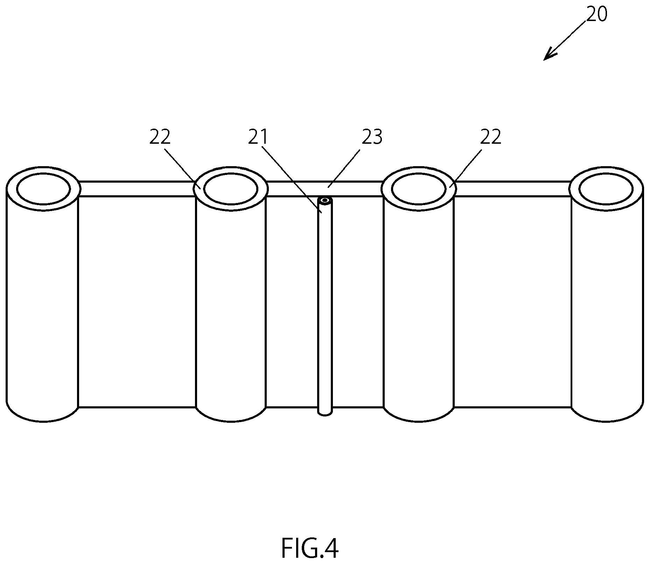

For example, a thermocouple, an optical fiber, an infrared camera, or the like can be employed as the heat transfer tube temperature measuring means 2, regardless of kind. FIG. 4 illustrates how an optical fiber 21 for measuring temperature is laid on a membrane panel 20 of a boiler. The optical fiber 21 is laid on a fin 23 connecting cooling tubes (heat transfer tube) 22 of the membrane panel 20. Thereby, the heat transfer tube temperature can be measured continuously along the axial direction of the cooling tube 22.

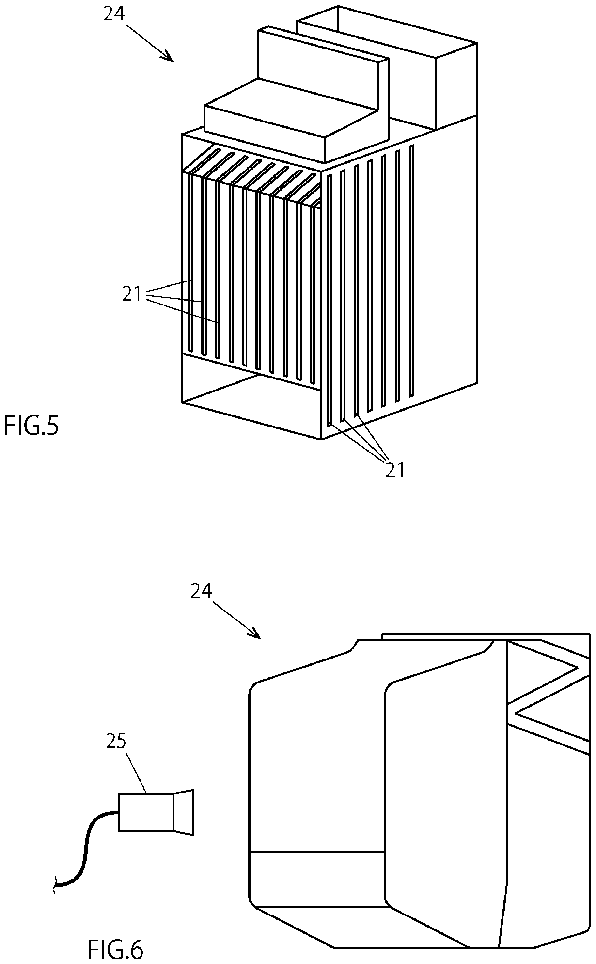

Also, as illustrated in FIG. 5, the heat transfer tube temperature can be measured over the entire surface of the furnace by laying the optical fibers 21 for measuring temperature over the entire surface of the furnace of a boiler 24. Alternatively, as illustrated in FIG. 6, the temperature may be measured over the entire surface of the furnace of the boiler 24 using an infrared camera 25.

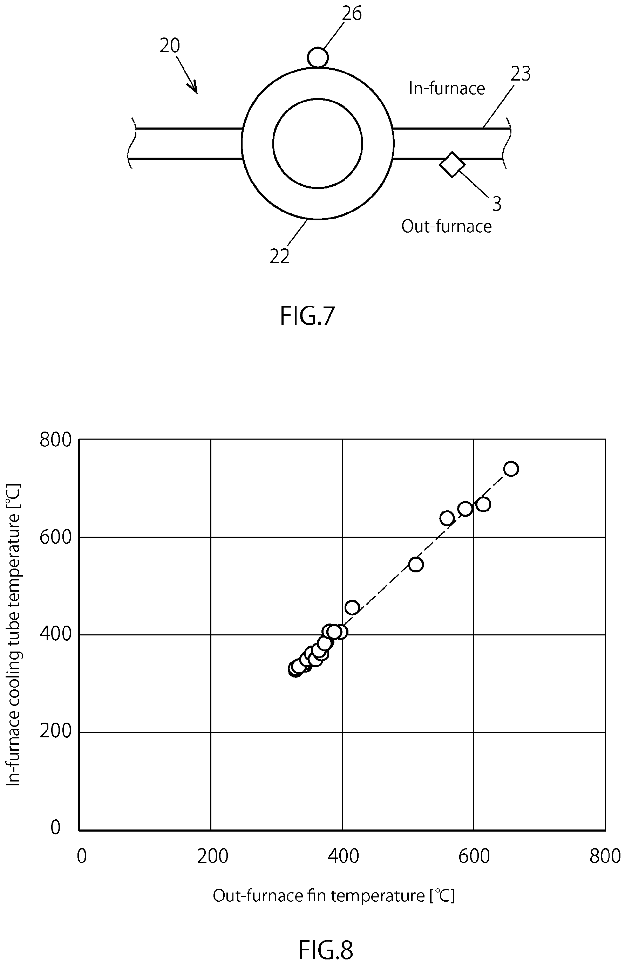

The heat transfer tube temperature measuring means in the example includes an out-furnace fin temperature measuring means 3 composed of an optical fiber provided on an out-furnace side outer wall surface of the fin 23 of the membrane panel 20 or the like, and an in-furnace cooling tube temperature calculating means 4 for calculating an in-furnace cooling tube temperature (temperature of the position of a reference sign 26 in FIG. 7) as a temperature of an in-furnace side outer wall surface of the cooling tube 22 based on an out-furnace fin temperature measured by the out-furnace fin temperature measuring means 3, as illustrated in FIG. 3 and FIG. 7.

The inventor has executed a FEM analysis and found that the out-furnace fin temperature and the in-furnace cooling tube temperature of the membrane panel 20 of the boiler 24 are in a linear relationship. FIG. 8 illustrates an example of the relationship between the out-furnace fin temperature and the in-furnace cooling tube temperature. The out-furnace fin temperature and the in-furnace cooling tube temperature increase resulted from the accumulation of scale in the cooling tube 22 or the like, and it is found that the relationship between the both is linear.

Accordingly, when the relationship between the in-furnace cooling tube temperature and the out-furnace fin temperature as illustrated in FIG. 8 is previously obtained according to the type, the in-furnace cooling tube temperature can be obtained from a measured value of the out-furnace fin temperature without measuring the in-furnace cooling tube temperature directly.

Thus, the temperature can be measured not in the furnace but out of the furnace, and therefore the level of heat resistance required for a thermometer can be lessened. Also, the measured data has higher long-term reliability, and higher stability compared to the in-furnace measurement.

The temperature of the cooling tube 22 increases as scale adheres and the scale thickness increases, for example, and the scale increases as the operation time of the boiler goes by, as mentioned above. Therefore, the elapsed time of boiler operation and the increased temperature of the cooling tube 22 are in the correspondence relationship.

In contrast, the heat transfer performance in scale differs depending on the thickness, adhering speed, density, and heat conductivity of the scale, and therefore the temperature increase of the cooling tube 22 cannot be uniquely obtained by an apparent operation time.

However, the curved shape of the time change curve of scale thickness does not substantially change, unless the operational condition of the boiler changes. Namely, even when sites or operational conditions differ, the horizontal axis (time axis) of the chart is only extended or contracted, and the curved shape does not substantially change.

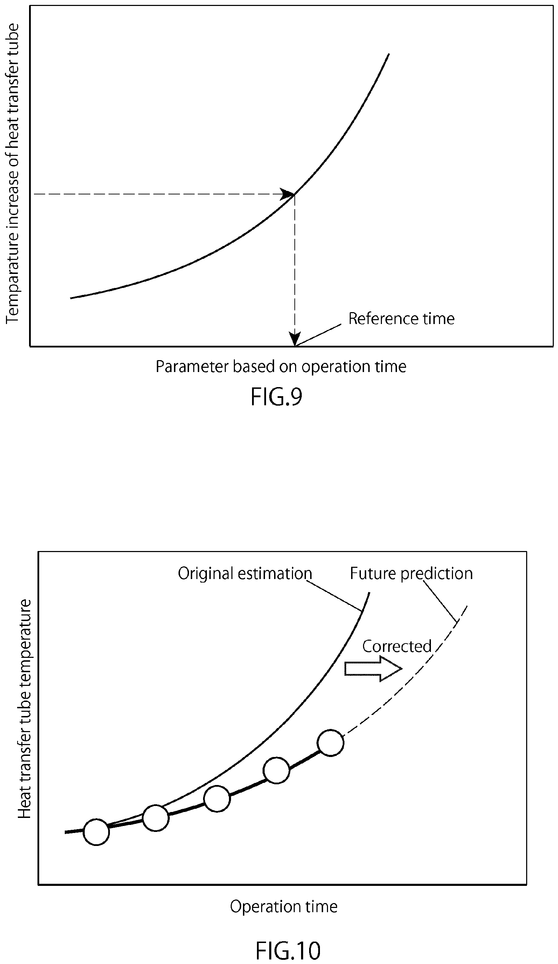

As a result, the temperature of the cooling tube 22 increases in a manner that the horizontal axis (time axis) of the chart is only extended and contracted and the curved shape does not substantially change when the operational condition of the boiler does not change, and the change corresponding to a cumulative time of the boiler's operation time increases with the same inclination (FIG. 9) .

The temperature of the heat transfer tube 22 at the present time is calculated by the calculation (the heat transfer tube temperature measuring means 2 in FIG. 3) using the temperature of the measured out-furnace fin 23, and the chart horizontal axis in the time change curve of the heat transfer tube 22 at the present time, which has been calculated, can be corrected (calibration).

A heat transfer tube temperature time change curve calibrating means 7-1 in FIG. 3 calibrates the data (base line curve) of the heat transfer tube temperature time change curve obtained from a heat transfer tube temperature time change curve reference database 8-1 according to the above-mentioned method. Namely, as the reference time in a parameter based on the operation time can be calculated from the heat transfer tube temperature as illustrated in FIG. 9, the chart horizontal axis in the heat transfer tube temperature time change curve is corrected as illustrated in FIG. 10 based on the reference time. As the time change curve of the heat transfer tube is corrected, a future heat transfer tube temperature can be predicted precisely.

A heat transfer tube temperature transition predicting means 9 in FIG. 3 predicts transition of the heat transfer tube temperature based on the calibrated equivalent heat transfer coefficient time change curve in FIG. 10.

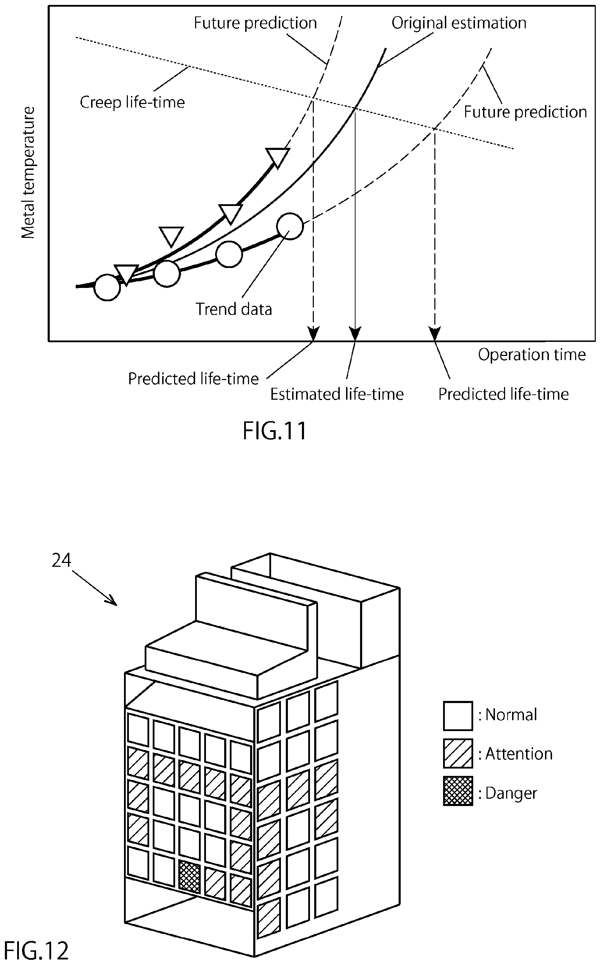

A creep life-time predicting means 10 in FIG. 3 calculates the creep damage quantity by a temperature history based on the transition predicting data of the heat transfer tube temperature obtained by the heat transfer tube temperature transition predicting means 9 so as to predict the creep life-time of the heat transfer tube, as illustrated in FIG. 11.

The prediction result of the creep life-time of each boiler location obtained by the creep life-time predicting means 10 can be displayed with high visibility as a contour or the like, as illustrated in FIG. 12.

As mentioned above, according to the heat transfer tube life estimating system 1 according to the example, transition in the future heat transfer tube temperature can be predicted using the temperature of the heat transfer tube measured in real time so as to precisely predict the creep life-time in real time based on the temperature transition.

Also, as the metal surface temperature of the heat transfer tube can be measured in real time so as to sequentially correct the time change curve of the equivalent heat transfer coefficient based on its measured temperature, the prediction performance of the future metal surface temperature of the heat transfer tube is improved.

Next, the heat transfer tube life estimating system according to an embodiment of the present invention will be described referring to figures. In the description of the embodiment, different/additional parts from/to the example are mainly described, and overlapped parts are sometimes omitted or simplified.

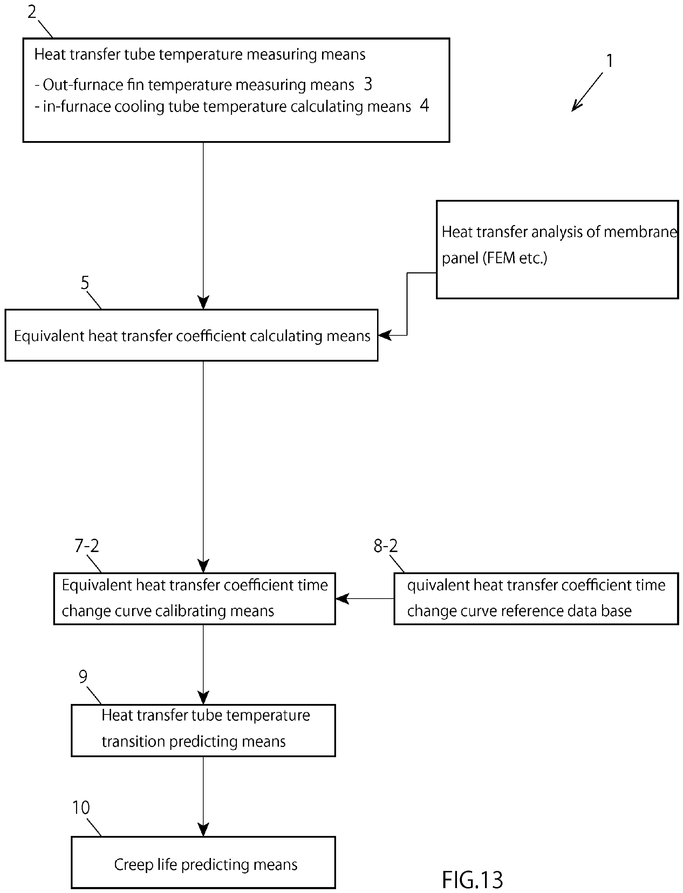

As illustrated in FIG. 13, a heat transfer tube life estimating system 1 according to the embodiment comprises a heat transfer tube temperature measuring means 2 for measuring a heat transfer tube temperature as a temperature of an outer wall surface of a heat transfer tube.

The thermocouple, the optical fiber, the infrared camera, or the like, for example can be used as the heat transfer tube temperature measuring means 2, like the example.

The heat transfer tube temperature measuring means 2 in the embodiment also includes the out-furnace fin temperature measuring means 3 composed of an optical fiber provided on the out-furnace side outer wall surface of the fin 23 of the membrane panel 20 or the like, and the in-furnace cooling tube temperature calculating means 4 for calculating the in-furnace cooling tube temperature (temperature of the position of the reference sign 26 in FIG. 7) as the temperature of the in-furnace side outer wall surface of the cooling tube 22 based on the out-furnace fin temperature measured by the out-furnace fin temperature measuring means 3, as illustrated in FIG. 13 and FIG. 7.

Thus, also in the embodiment, the temperature can be measured not in the furnace but out of the furnace, and therefore the level of heat resistance required for a thermometer can be lessened. Also, the measured data has higher long-term reliability, and higher stability compared to the in-furnace measurement.

In addition, the heat transfer performance of a heat transfer tube such as the cooling tube 22 of the membrane panel 20, an overheating tube, or the like, can be expressed as an equivalent heat transfer coefficient *he* regarding the apparent heat transfer (including the heat conductivity of scale) between the wafer flowing through the tube and the tube surface.

Here, if the water temperature in the cooling tube 22 of the membrane panel 20 is assumed to be the saturation temperature, the relationship between the metal surface temperature of the heat transfer tube and the equivalent heat transfer coefficient *he* can be calculated by finite element analysis, or the like, and the relationship between the both can be previously grasped.

Accordingly, when the metal surface temperature of the heat transfer tube is measured, the equivalent heat transfer coefficient *he* can be estimated from the previously grasped relationship between the metal surface temperature of the heat transfer tube and the equivalent heat transfer coefficient *he.*

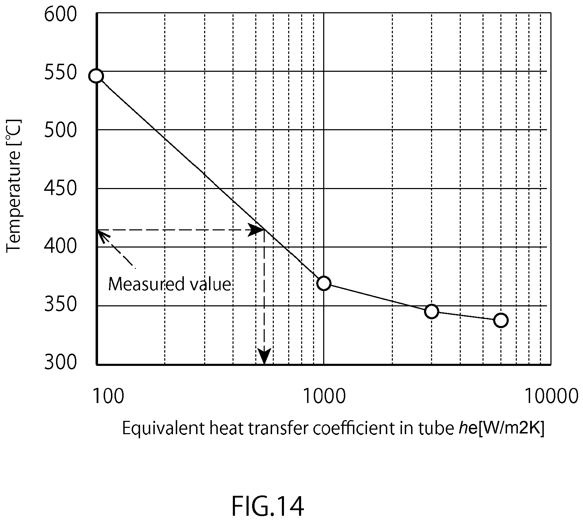

The equivalent heat transfer coefficient calculating means 5 in FIG. 13 calculates the equivalent heat transfer coefficient *he* by the above-described method. Namely, as illustrated in FIG. 14, the equivalent heat transfer coefficient calculating means 5 calculates the equivalent heat transfer coefficient *he* inside the heat transfer tube based on the heat transfer tube temperature as a temperature of the outer wall surface of the heat transfer tube.

Note that, the equivalent heat transfer coefficient *he* as an apparent heat transfer can be determined mainly by the heat conductivity and thickness of scale. The heat conductivity of scale is different in each site because it is affected by kinds of boilers, water quality, production temperature, or the like. However, here the scale thickness and the equivalent heat transfer coefficient *he* correspond one-to-one, provided that an average (proper) value as the heat conductivity of scale is tentatively determined based on the experience.

With regard to the change in scale thickness with time, although the scale growth speed is changed by sites, operational conditions, water quality, or the like, the curved shape does not substantially change if the operational condition of the boiler does not change. Namely, even when sites or operational conditions are different, the horizontal axis of the chart simply extends or contracts and the curved shape does not substantially change.

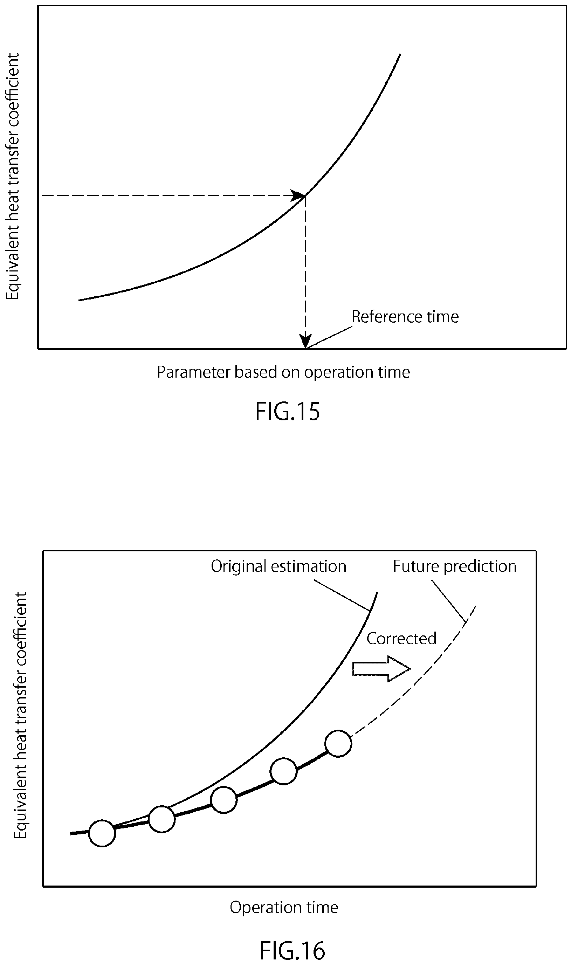

Accordingly, the equivalent heat transfer coefficient *he* also increases with the same inclination (FIG. 15) regarding the change in relation to the cumulative time of boiler operation as with the time change in scale thickness (scale growth speed), provided that the boiler operation condition does not change.

The value of the equivalent heat transfer coefficient at the present time is calculated based on FIG. 14 using the measured heat transfer tube temperature, and the chart horizontal axis in the time change curve of the measured equivalent heat transfer coefficient at the present time can be corrected (calibrated).

The equivalent heat transfer coefficient time change curve calibrating means 7-2 in FIG. 13 calibrates the data of the equivalent heat transfer coefficient time change curve (base line curve) obtained from the equivalent heat transfer coefficient time change curve reference data base 8-2 according to the above-mentioned method. Namely, as the reference time in a parameter based on the operation time can be calculated from the equivalent heat transfer coefficient as illustrated in FIG. 15, the chart horizontal axis in the equivalent heat transfer coefficient time change curve is corrected as illustrated in FIG. 16 based on the reference time. As the time change curve of the equivalent heat transfer coefficient has been corrected, a future equivalent heat transfer coefficient can be predicted accurately.

The heat transfer tube temperature transition predicting means 9 in FIG. 13 predicts transition in the heat transfer tube temperature based on the calibrated equivalent heat transfer coefficient time change curve in FIG. 16.

The creep life-time predicting means 10 in FIG. 13 calculates the creep damage quantity by the temperature history based on the transition predicting data of the heat transfer tube temperature obtained by the heat transfer tube temperature transition predicting means 9 so as to predict the creep life-time of the heat transfer tube, as illustrated in FIG. 16.

The prediction result of the creep life-time of each the boiler location obtained by the creep life-time predicting means 10 can also be displayed with a high visibility as a contour or the like, as illustrated in FIG. 12.

As described above, according to the heat transfer tube life estimating system 1 according to the embodiment, transition in a future equivalent heat transfer coefficient *he* can be predicted using the temperature of the heat transfer tube measured in real time, and how a future metal surface temperature of the heat transfer tube is transited by the relationship between the metal surface temperature of the heat transfer tube obtained from finite element analysis or the like and the equivalent heat transfer coefficient *he* is predicted and also the creep life-time is accurately predicted in real time based on the temperature transition.

In addition, as the metal surface temperature of the heat transfer tube is measured in real time and the time change curve of the equivalent heat transfer coefficient can be sequentially corrected based on the measured temperature, the prediction performance of a future metal surface temperature of the heat transfer tube is improved.

Next, the heat transfer tube life estimating system according to another example not covered by the present invention will be described referring to the figures. In the description of the example, different/additional parts from/to the first example or the embodiment are mainly described, and contents in common with the first example or the embodiment are sometimes omitted or simplified.

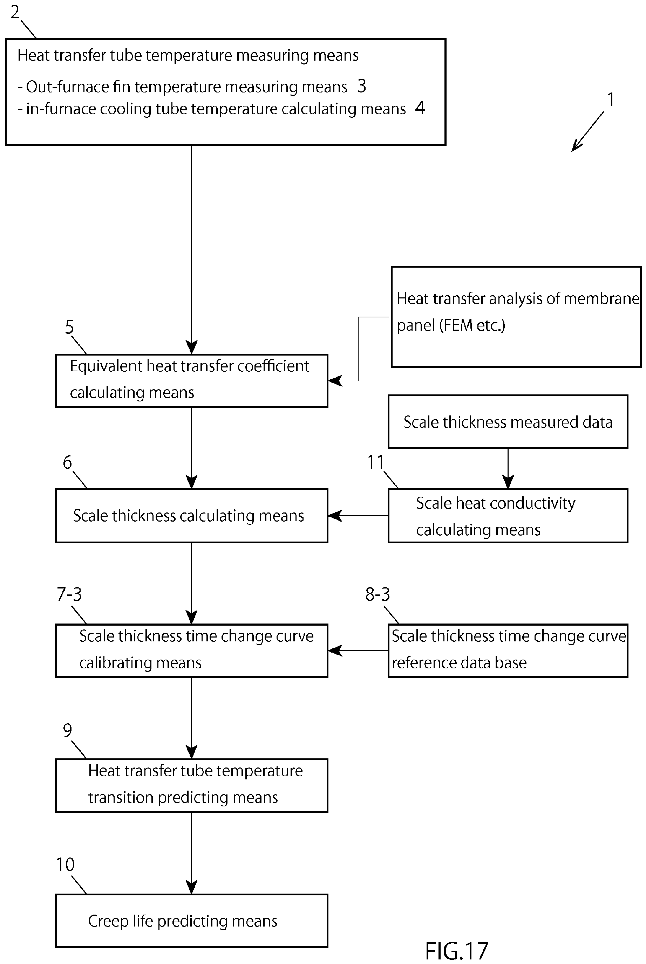

As previously mentioned, the scale thickness calculating means 6 in FIG. 17 calculates the scale thickness using the equivalent heat transfer coefficient calculated by the same method as the embodiment by utilizing that the scale thickness and the equivalent heat transfer coefficient *he* are in a one-to-one relationship and the scale thickness is uniquely defined by the equivalent heat transfer coefficient. Namely, the scale thickness in the heat transfer tube is calculated based on the equivalent heat transfer coefficient *he* obtained by the equivalent heat transfer coefficient calculating means 5.

The time change curve of scale thickness continuously changes, provided that the operational condition of the boiler does not change. As for the time change curve of the scale thickness, the scale growth speed changes according to sites, operational conditions, water quality, or the like, however, the curve shape does not substantially change. Namely, the horizontal axis of the chart simply extends or contracts.

At the time point, the scale thickness at the present time has been estimated, and therefore the chart horizontal axis in the time change curve of scale thickness can be corrected (calibrated).

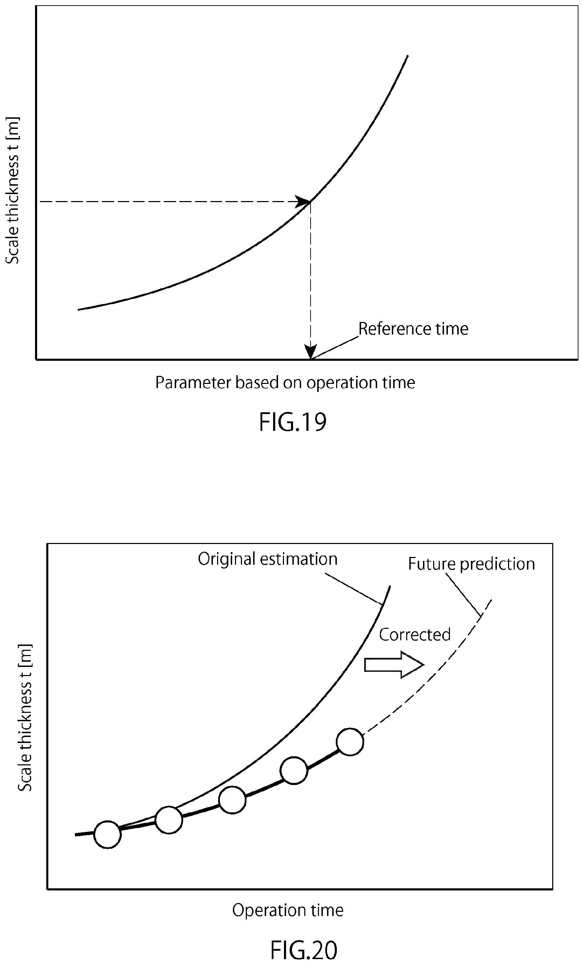

A time change curve calibrating means 7-3 in FIG. 17 calibrates data of a scale thickness time change curve (base line curve) obtained from a scale thickness time change curve reference data base 8-3 according to the above-mentioned method. Namely, as the reference time in a parameter based on the operation time can be calculated from the scale thickness as illustrated in FIG. 19, the chart horizontal axis in the scale thickness time change curve is corrected as illustrated in FIG. 20 based on the reference time. As the time change curve of the scale thickness has been corrected, a future scale thickness can be predicted accurately.

The heat transfer tube temperature transition predicting means 9 in FIG. 17 predicts transition in the heat transfer tube temperature based on the calibrated scale thickness time change curve in FIG. 20.

The creep life-time predicting means 10 in FIG. 17 calculates the creep damage quantity by the temperature history based on the transition predicting data of the heat transfer tube temperature obtained by the heat transfer tube temperature transition predicting means 9 so as to predict the creep life-time of the heat transfer tube, as illustrated in FIG. 11.

The prediction result of the creep life-time of each the boiler location obtained by the creep life-time predicting means 10 can also be displayed with a high visibility as a contour or the like, as illustrated in FIG. 12.

As described above, according to the heat transfer tube life estimating system 1 according to the second example, transition in a future equivalent heat transfer coefficient *he* can be predicted from the relationship between the scale thickness and the equivalent heat transfer coefficient *he,* and how a future metal surface temperature of the heat transfer tube is transited by the relationship between the metal surface temperature of the heat transfer tube obtained from finite element analysis or the like and the equivalent heat transfer coefficient *he* is predicted and also the creep life-time is accurately predicted in real time based on the temperature transition.

In addition, the time change curve of the scale thickness can be sequentially corrected by repeating the measurement of the metal surface temperature of the heat transfer tube, and therefore the prediction performance of a future metal surface temperature of the heat transfer tube is improved.

Note that, although the heat conductivity of scale is considered by tentatively determining an average (proper) value as described above, the scale thickness is sometimes measured in a periodic inspection of the boiler.

Then, when an actually measured value of the scale thickness is obtained in the periodic inspection, a heat transfer coefficient of scale is determined according to the following procedure by the scale heat conductivity calculating means 11 in FIG. 17,

- The equivalent heat transfer coefficient *he* in the tube is calculated from the metal temperature of the heat transfer tube measured in the operation immediately before the periodic inspection.
- The actually measured scale thickness and the calculated equivalent heat transfer coefficient *he* are plotted on their relationship diagram.
- The heat transfer coefficient is determined so that a line indicating the relationship between the scale thickness and the equivalent heat transfer coefficient *he* passes through the plot.

As the heat transfer coefficient of scale in the target site is measured according to the above, the prediction accuracy of a future metal surface temperature of the heat transfer tube is improved further.

As stated above, by the heat transfer tube life estimating system according to the embodiment, the life of the heat transfer tube of the boiler can be estimated accurately in real time while continuing to operate.

As the boiler equipped with the system can previously grasp the timing of reaching the life of the heat transfer tube, maintenance and repair can be performed systematically.

In addition, by displaying the estimation result of the creep life-time of the heat transfer tube on the contour, the distribution of dangerous states can be visually informed immediately.

Furthermore, by measuring the heat transfer tube temperature over a wide range, an abnormality generating portion difficult to be predicted beforehand can also be coped with in addition to the life estimation within the conventional prediction range.

Therefore, it is possible to minimize the operation stop period even when installed in a remote site such as on the sea and so an urgent repair is difficult.

Reference Signs List

1 ... heat transfer tube life estimating system
2 ... heat transfer tube temperature measuring means
3 ... out-furnace fin temperature measuring means
4 ... in-furnace cooling tube temperature calculating means
5 ... equivalent heat transfer coefficient calculating means
6 ... scale thickness calculating means
7-1 ... heat transfer tube temperature time change curve carbureting means
7-2 ... equivalent heat transfer coefficient time change curve calibrating means
7-3 ... scale thickness time change curve calibrating means
8-1 ... heat transfer tube temperature time change curve reference data base
8-2 ... equivalent heat transfer coefficient time change curve reference data base
8-3 ... scale thickness time change curve reference data base
9 ... heat transfer tube temperature transition predicting means
10 ... creep life-time predicting means
11 ... scale heat conductivity calculating means 20 ... membrane panel
21 ... optical fiber
22 ... cooling tube
23 ... fin
24 ... boiler
25 ... infrared camera
26 ... measuring position of in-furnace cooling tube temperature

**Claims**

1. A heat transfer tube life estimating system (1) for estimating a life of a heat transfer tube of a boiler (24), the system comprising:

   an equivalent heat transfer coefficient calculating unit (5) configured to calculate an equivalent heat transfer coefficient $h_e$ inside of the heat transfer tube based on a heat transfer tube temperature as a temperature of an outer wall surface of the heat transfer tube $Tt_{22}$ [K] ;
   an equivalent heat transfer coefficient time change curve calibrating unit (7-2) configured to calibrate an equivalent heat transfer coefficient time change curve representing a change in the equivalent heat transfer coefficient $h_e$ with time based on the equivalent heat transfer coefficient $h_e$;
   a heat transfer tube temperature transition predicting unit (9) configured to predict a transition in the heat transfer tube temperature based on the equivalent heat transfer coefficient time change curve which has been calibrated; and
   a creep life-time predicting unit (10) configured to predict a creep life-time of the heat transfer tube based on a transition predicting data of the heat transfer tube temperature obtained by the heat transfer tube temperature transition predicting unit (9);

wherein a heat conductivity of scale $\lambda_2$ [w/m · K] is constant, a temperature difference inside scale $\Delta T$ [K] increases and the temperature of an outer wall surface of the heat transfer tube $Tt_{22}$ [K] rises according to increase of a thickness of scale $t_2$ [m] ,
a scale surface heat conductivity is $h_2$ [W/m² · K] , and
the equivalent heat transfer coefficient $h_e$ is represented by a formula

$$h_e = 1/(t_2/\lambda_2 + 1/h_2) \ [W/m^2 \cdot K]$$

, and
wherein a fluid temperature is Tw [K] , a heat flux q [W/m²] is represented by a formula q= $h_e\Delta T$, wherein $\Delta T$= $Tt_{22}$-Tw [K] .

2. The heat transfer tube life estimating system (1) according to claim 1,

wherein the heat transfer tube is a cooling tube (22) of a membrane panel (20) configuring a furnace wall of the boiler (24),
the life estimating system (1) further comprising:

an out-furnace fin temperature measuring unit (3) configured to measure an out-furnace fin temperature as a temperature of an out-furnace side outer wall surface of a fin of the membrane panel; and
an in-furnace cooling tube temperature calculating unit (4) configured to calculate an in-furnace cooling tube temperature as a temperature of an in-furnace side outer wall surface of the cooling tube based on the out-furnace fin temperature, and
wherein the in-furnace cooling tube temperature is used as the heat transfer tube temperature.

3. The heat transfer tube life estimating system (1) according to any one of claims 1 to 2, wherein the heat transfer tube temperature is continuously obtained along an axial direction of the heat transfer tube.

4. The heat transfer tube life estimating system (1) according to any one of claims 1 to 3, wherein the heat transfer tube temperature is obtained over an entire surface of a furnace wall of the boiler (24).

5. The heat transfer tube life estimating system (1) according to claim 3 or 4, wherein the heat transfer tube temperature is obtained using an optical fiber (21) or an infrared camera (25).

**Patentansprüche**

1. System (1) zur Lebensdauerschätzung eines Wärmeübertragungsrohrs zum Schätzen einer Lebensdauer eines Wärmeübertragungsrohrs eines Boilers (24), wobei das System umfasst:

eine Einheit (5) zur Berechnung des äquivalenten Wärmedurchgangskoeffizienten, die dazu ausgestaltet ist, einen äquivalenten Wärmedurchgangskoeffizienten $h_e$ im Inneren des Wärmeübertragungsrohrs basierend auf einer Wärmeübertragungsrohrtemperatur als eine Temperatur einer Außenwandfläche des Wärmeübertragungsrohrs $Tt_{22}$ [K] zu berechnen;
eine Einheit (7-2) zur Kalibrierung einer Kurve der zeitlichen Veränderung des äquivalenten Wärmedurchgangskoeffizienten, die dazu ausgestaltet ist, eine Kurve der zeitlichen Veränderung des äquivalenten Wärmedurchgangskoeffizienten zu kalibrieren, die eine Änderung des äquivalenten Wärmedurchgangskoeffizienten $h_e$ mit der Zeit basierend auf dem äquivalenten Wärmedurchgangskoeffizienten $h_e$ darstellt;
eine Einheit (9) zur Vorhersage des Wärmeübertragungsrohr-Temperaturübergangs, die dazu ausgestaltet ist, einen Übergang bei der Temperatur des Wärmeübertragungsrohrs basierend auf der Kurve der zeitlichen Veränderung des äquivalenten Wärmedurchgangskoeffizienten, die kalibriert wurde, vorherzusagen; und
eine Einheit (10) zur Vorhersage der Kriechlebensdauer, die dazu ausgestaltet ist, eine Kriechlebensdauer des Wärmeübertragungsrohrs basierend auf Übergangsvorhersagedaten der
Wärmeübertragungsrohrtemperatur vorherzusagen, die durch die Einheit (9) zur Vorhersage des Wärmeübertragungsrohr-Temperaturübergangs erhalten wird;
wobei eine Wärmeleitfähigkeit des Maßstabs $\lambda_2$ [W/m · K] konstant ist, eine Temperaturdifferenz innerhalb des Maßstabs $\Delta T$[K] zunimmt und die Temperatur einer äußeren Wandfläche des Wärmeübertragungsrohrs $Tt_{22}$

[K] gemäß einer Zunahme einer Dicke des Maßstabs $t_2$ [m] steigt,
ein Maßstab für die Flächenwärmeleitfähigkeit $h_2$ [W/m$^2 \cdot$ K] ist, und
der äquivalente Wärmedurchgangskoeffizient $\boldsymbol{h_e}$ durch eine folgende Formel dargestellt wird:

$$\boldsymbol{h_e} = 1/(t_2/ \lambda_2 + 1/h_2) \ [\text{W/m}^2 \cdot \text{K}]$$

, und
wobei eine Fluidtemperatur Tw [K] ist und ein Wärmefluss q[W/m$^2$] durch eine Formel q = $\boldsymbol{h_e}\Delta$T dargestellt wird,
wobei $\Delta$T=Tt$_{22}$-Tw[K].

2. System (1) zur Lebensdauerschätzung eines Wärmeübertragungsrohrs nach Anspruch 1,

wobei das Wärmeübertragungsrohr ein Kühlrohr (22) einer Membrantafel (20) ist, die eine Ofenwand des Boilers (24) ausgestaltet,
wobei das System (1) zur Lebensdauerschätzung ferner umfasst:

eine Einheit (3) zur Messung einer Ofenaußenrippentemperatur, die dazu ausgestaltet ist, eine Temperatur einer Ofenaußenrippe als eine Temperatur einer äußeren Wandfläche einer Ofenaußenseite einer Rippe der Membrantafel zu messen; und
eine Einheit (4) zur Berechnung der Temperatur eines Offeninnenkühlrohrs, die dazu ausgestaltet ist, eine Temperatur eines Ofeninnenkühlrohrs als eine Temperatur einer äußeren Wandoberfläche einer Ofeninnenseite des Kühlrohrs basierend auf der Ofenaußenrippentemperatur zu berechnen, und
wobei die Temperatur des Ofeninnenkühlrohrs als die Wärmeübertragungsrohrtemperatur verwendet wird.

3. System (1) zur Lebensdauerschätzung eines Wärmeübertragungsrohrs nach einem der Ansprüche 1 bis 2, wobei die Wärmeübertragungsrohrtemperatur kontinuierlich entlang einer axialen Richtung des Wärmeübertragungsrohrs erhalten wird.

4. System (1) zur Lebensdauerschätzung eines Wärmeübertragungsrohrs nach einem der Ansprüche 1 bis 3, wobei die Wärmeübertragungsrohrtemperatur über eine gesamte Fläche einer Ofenwand des Boilers (24) erhalten wird.

5. System (1) zur Lebensdauerschätzung eines Wärmeübertragungsrohrs nach Anspruch 3 oder 4, wobei die Wärmeübertragungsrohrtemperatur unter Verwendung einer optischen Faser (21) oder einer Infrarotkamera (25) erhalten wird.

## Revendications

1. Système d'estimation de durée de vie de tube de transfert de chaleur (1) pour estimer une durée de vue d'un tube de transfert de chaleur d'une chaudière (24), le système comprenant :

une unité de calcul de coefficient de transfert de chaleur équivalent (5) configurée pour calculer un coefficient de transfert de chaleur équivalent $\boldsymbol{h_e}$ à l'intérieur du tube de transfert de chaleur sur la base d'une température de tube de transfert de chaleur en tant qu'une température d'une surface de paroi extérieure du tube de transfert de chaleur Tt$_{22}$ [K] ;
une unité d'étalonnage de courbe de changement dans le temps de coefficient de transfert de chaleur équivalent (7-2) configurée pour étalonner une courbe de changement dans le temps de coefficient de transfert de chaleur équivalent représentant un changement dans le temps du coefficient de transfert de chaleur équivalent $\boldsymbol{h_e}$ sur la base du coefficient de transfert de chaleur équivalent $\boldsymbol{h_e}$ ;
une unité de prédiction de transition de température de tube de transfert de chaleur (9) configurée pour prédire une transition de la température de tube de transfert de chaleur sur la base de la courbe de changement dans le temps de coefficient de transfert de chaleur équivalent qui a été étalonnée ; et
une unité de prédiction de longévité de fluage (10) configurée pour prédire une longévité de fluage du tube de transfert de chaleur sur la base de données de prédiction de transition de la température de tube de transfert de chaleur obtenue par l'unité de prédiction de transition de température de tube de transfert de chaleur (9) ;
dans lequel une conductivité thermique de tartre $\lambda_2$ [W/m $\cdot$ K] est constante, une différence de température dans le tartre $\Delta$T [K] augmente et la température d'une surface de paroi extérieure du tube de transfert de

chaleur $Tt_{22}$ [K] monte en fonction d'une augmentation d'une épaisseur de tartre $t_2$ [m],
une conductivité thermique de surface de tartre est $h_2$ [W/m$^2 \cdot$ K], et
le coefficient de transfert de chaleur équivalent $\mathit{h_e}$ est représenté par la formule suivante :

$$\mathit{h_e} = 1/(t_2/\ \lambda_2 + 1/h_2)\ [\text{W/m}^2 \cdot \text{K}]$$

, et
dans lequel une température de fluide est Tw [K], un flux de chaleur q [W/m$^2$] est représenté par une formule
$q = \mathit{h_e}\Delta T$, où $\Delta T = Tt_{22} - Tw$[K],

2. Système d'estimation de durée de vie de tube de transfert de chaleur (1) selon la revendication 1,

dans lequel le tube de transfert de chaleur est un tube de refroidissement (22) d'un panneau de membrane (20) configurant une paroi de foyer de la chaudière (24),
le système d'estimation de durée de vie (1) comprenant en outre :

une unité de mesure de température d'ailette de sortie de foyer (3) configurée pour mesurer une température d'ailette de sortie de foyer en tant qu'une température d'une surface de paroi extérieure de côté de sortie de foyer d'une ailette du panneau de membrane ; et
une unité de calcul de température de tube de refroidissement d'entrée de foyer (4) configurée pour calculer une température de tube de refroidissement d'entrée de foyer en tant qu'une température d'une surface de paroi extérieure de côté d'entrée de foyer du tube de refroidissement sur la base de la température d'ailette de sortie de foyer, et
dans lequel la température de tube de refroidissement d'entrée de foyer est utilisée en tant que la température de tube de transfert de chaleur.

3. Système d'estimation de durée de vie de tube de transfert de chaleur (1) selon la revendication 1 ou 2, dans lequel la température de tube de transfert de chaleur est obtenue en continu le long d'une direction axiale du tube de transfert de chaleur.

4. Système d'estimation de durée de vie de tube de transfert de chaleur (1) selon l'une quelconque des revendications 1 à 3, dans lequel la température de tube de transfert de chaleur est obtenue sur une surface complète d'une paroi de foyer de la chaudière (24).

5. Système d'estimation de durée de vie de tube de transfert de chaleur (1) selon la revendication 3 ou 4, dans lequel la température de tube de transfert de chaleur est obtenue en utilisant une fibre optique (21) ou une caméra infrarouge (25).

Heat transfer tube wall heat transfer coefficient

Scale heat transfer coefficient

Boiler combustion chamber (boiler inside)

$\lambda 1$

$\lambda 2$

Inside of heat transfer tube (flow channel)

Combustion gas

Further increase of scale

Temperature Tg

Adhesion of scale to tube inner wall surface

Tt13

Heat transfer tube surface temperature rising due to scale adhesion

Tt12

Tt23

Water/Steam

Tt22

Tube outer wall surface heat transfer coefficient h1

Tt11

Ts3

Heat transfer tube surface temperature

Tt21

Ts2

Tube inner wall surface heat transfer coefficient h2

Temperature Tw

Tube inner wall surface heat transfer coefficient h2

t1

t2

Relation between scale thickness and heat transfer (heat transfer tube surface temperature)

FIG.1

EP 3 098 508 B1

(a)

(b)

FIG.2

1

2

Heat transfer tube temperature measuring means

- Out-furnace fin temperature measuring means  3
- in-furnace cooling tube temperature calculating means  4

7-1

Heat transfer tube temperature
time change curve calibrating means

8-1

Heat transfer tube temperature time
change curve reference database

9

Heat transfer tube temperature
transition predicting means

10

Creep life predicting means

FIG.3

FIG.4

24

21

21

FIG.5

24

25

FIG.6

FIG.7

Out-furnace fin temperature [℃]

FIG.8

Temparature increase of heat transfer tube

Reference time

Parameter based on operation time

FIG.9

Heat transfer tube temperature

Original estimation     Future prediction

Corrected

Operation time

FIG.10

FIG.11

FIG.12

2

```
Heat transfer tube temperature measuring means

- Out-furnace fin temperature measuring means  3
- in-furnace cooling tube temperature calculating means  4
```

1

```
Heat transfer analysis of membrane
panel (FEM etc.)
```

5

```
Equivalent heat transfer coefficient calculating means
```

7-2

```
Equivalent heat transfer coefficient time
change curve calibrating means
```

8-2

```
quivalent heat transfer coefficient time
change curve reference data base
```

9

```
Heat transfer tube temperature
transition predicting means
```

10

```
Creep life predicting means
```

FIG.13

FIG.14

FIG.15

FIG.16

2

Heat transfer tube temperature measuring means

- Out-furnace fin temperature measuring means  3
- in-furnace cooling tube temperature calculating means  4

1

5

Equivalent heat transfer coefficient calculating means

Heat transfer analysis of membrane panel (FEM etc.)

Scale thickness measured data

6

Scale thickness calculating means

11

Scale heat conductivity calculating means

7-3

Scale thickness time change curve calibrating means

8-3

Scale thickness time change curve reference data base

9

Heat transfer tube temperature transition predicting means

10

Creep life predicting means

FIG.17

FIG.18

Scale thickness t [m]

Reference time

Parameter based on operation time

FIG.19

Original estimation          Future prediction

Corrected

Scale thickness t [m]

Operation time

FIG.20

**EP 3 098 508 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002022686 A